(19) Europäisches Patentamt
European Patent Office
Office européen des brevets

(11) Publication number: 0 070 013
B1

(12) # EUROPEAN PATENT SPECIFICATION

(45) Date of publication of patent specification: 15.10.86

(21) Application number: 82106172.8

(22) Date of filing: 12.07.82

(51) Int. Cl.⁴: C 07 D 501/04, C 07 D 501/57, C 07 D 499/00, A 61 K 31/00, C 07 D 317/40, C 07 F 9/40, C 07 D 405/12

(54) **Oral absorption enhancement of carboxylic acid pharmaceuticals using (5-alkyl-2-oxo-1,3-dioxolen-4-yl)methyl ester group.**

(30) Priority: 13.07.81 US 282821
20.07.81 US 285170
25.11.81 US 324728
01.02.82 US 344413
06.04.82 US 366036

(43) Date of publication of application:
19.01.83 Bulletin 83/03

(45) Publication of the grant of the patent:
15.10.86 Bulletin 86/42

(84) Designated Contracting States:
AT BE CH DE FR GB IT LI LU NL SE

(56) References cited:
EP-A-0 039 086
EP-A-0 039 477

(73) Proprietor: MERCK & CO. INC.
126, East Lincoln Avenue P.O. Box 2000
Rahway New Jersey 07065 (US)

(72) Inventor: Burton, Christensen G.
770 Anderson Avenue Apt. 19H
Cliffside Park New Jersey 07010 (US)

(74) Representative: Abitz, Walter, Dr.-Ing. et al
Abitz, Morf, Gritschneder, Freiherr von
Wittgenstein Postfach 86 01 09
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

Summary of the invention

This invention provides a novel moiety

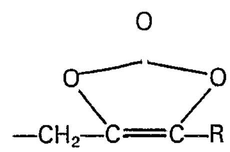

I

wherein R is alkyl of 1—6 carbon atoms, especially methyl or t-butyl; which when present as an ester of a carboxylic acid, i.e. cefoxitin, methyldopa or 2-(ethylthio)-6β-(1-R-hydroxyethyl)-penem-3-carboxylic acid, enhances oral absorbability of the ester product.

EP—A—39086 refers to (5-methyl-2-oxo-1,3-dioxolen-4-yl)-methylesters of ampicillin.

The use of 1,3-dioxolen-2-one derivatives as modifiers for prodrugs preparation is known from EP—A—39477. The only pharmaceutically active compound mentioned in this reference is ampicillin.

The useful esters of this invention are prepared by utilizing the alcohol or halide, e.g. chloride or bromide form of group I above in reaction with the free acid or salt form of the desired pharmaceutically active compound of which enhanced oral absorption is desired. Preferably, the bromide form:

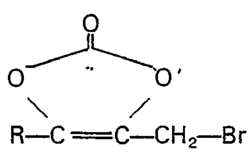

II

(the 5-methyl compound is prepared as described by H. Scherf and H. Plum, Liebiegs Ann, Chem., 1977, 27—32, and the other 5-alkyls, including 5-t-butyl, are prepared in a similar fashion) is reacted with either the lithium or silver salt form of the pharmaceutical acid.

Preferable reaction conditions include a ratio of from about equivalent to twice equivalent amounts of Compound II to the pharmaceutical acid, in a solvent such as hexamethylphosphoramide, dimethylformamide, acetonitrile, dimethyl sulfoxide, or the like, at from about 0—50°C, preferably about 25—35°C, for from 2—24 hours, (the reaction proceeds more quickly at the higher end of the range).

The ester can be recovered in from 10—60% yield, and is purified by recrystallization in an appropriate solvent system, such as ethylacetate-diethyl ether.

The preparation of the (5-methyl-2-oxo-1,3-dioxolen-4-yl)methyl ester of cefoxitin illustrates one embodiment of this invention.

Example 1

Step A. Preparation of cefoxitin lithium salt

To a stirred suspension of 42.75 g (0.1m) of cefoxitin in 1 liter of water is added at 0—5° over a period of 45—60 minutes a solution of 0.1N lithium hydroxide. The initial pH of the mixture is about 3.0. Addition of the alkaline solution is so regulated that the pH does not rise above 5.5. The solution, about 2.5 liters, is filtered to remove a small amount of undissolved free acid and then subdivided in equal portions into 5×5 liter round bottom flasks for lyophilization and for use in the next steps.

Step B. Esterification

Fifty-four grams (0.125 m) of cefoxitin Li salt is charged into a 3-liter, 3-necked round bottom flask, fitted with a stirrer and Drierite tube. The solid is stirred and 540 ml of hexamethylphosphoramide (HMPA), pH 6.7, is added. Upon the addition of (5-methyl-2-oxo-1,3-dioxolen-4-yl)methyl bromide, (0.25 m), the temperature rises slightly, and is kept at about room temperature, while stirring 20 minutes. Esterification is monitored by tlc (silica gel plates, ethylacetate-chloroform solvent).

After the end of the reaction period, the solution is added to a stirred mixture of 5 liters of water and 5 liters of ethyl acetate. The separated aqueous phase is extracted with 2×2 liters of ethyl acetate, and the combined solution is washed with 2×2 liters of water and 1×2 liters of saturated sodium chloride solution. The solution is dried over anhydrous magnesium sulfate, treated with 15 g of Darco G-60, (activated charcoal), and after filtration, concentrated under vacuum at room temperature. The concentrate is dissolved in 270 ml of methylene chloride, and the resulting solution added slowly to 13 liters of petroleum-benzin with rapid stirring. The precipitate is filtered off, washed with petroleum-benzin and dried at r.t./0.1 mm/18 hrs./$P_2O_5$.

Step C. Purification

The above impure ester is dissolved in 400 ml of ethyl acetate at room temperature. The solution is treated with 2×7.0 g of Darco G-60, washing the charcoal cake well after each treatment with ethyl acetate.

The combined filtrate and washes are concentrated at room temperature at 2—5 mm to a volume of about 500 ml. To the stirred concentrated solution under anhydrous conditions is added slowly 700 ml of ethyl ether to the point of producing a first permanent cloudiness. The solution is clarified with a few drops of ethyl acetate and then ether (*ca* 5 ml) is carefully added to cloudiness again. After stirring for a few minutes, the solution is allowed to stand undisturbed at room temperature for 3—4 hours, and then aged at 5° overnight. The mixture is filtered, and the solid washed with 3×50 ml of ether. The cefoxitin ester is dried at r.t./0.1 mm/$P_2O_5$/24 hours.

Alternatively, the silver salt of cefoxitin can be prepared by reacting 1 equivalent of cefoxitin free acid with 1 equivalent sodium bicarbonate in water then adding the resulting solution to a solution of 2 equivalents of silver nitrate in aqueous solution. The silver salt precipitates, is dried and used as the starting material in reaction with the (5-methyl-2-oxo-1,3-dioxolen-4-yl)methyl bromide, as described above, as a suspension in anhydrous acetonitrile.

The final product, (5-methyl-2-oxo-1,3-dioxolen-4-yl)methyl ester of cefoxitin can be administered to humans at the usual dosage levels and regimen as cefoxitin sodium, a widely available product. The product can be given IV or orally, at levels of 2—20 mg/kg/day. Blood levels of drug are enhanced in comparison with cefoxitin sodium.

Furthermore, methyldopa and 2-(ethylthio)-6β-(1R-hydroxyethyl)-penem-3-carboxylic acid can have enhanced blood levels, if they are employed in the form of the (5-alkyl-2-oxo-1,3-dioxolen-4-yl)-methyl ester. The ester form of these compounds is prepared using the same general procedure described above for the cefoxitin ester.

Cefoxitin methyldopa and 2-(ethylino)-6β-(1R-hydroxyethyl)-penem-3-carboxylic acid are known in the art as pharmaceuticals in the acid or sodium salt form. Their dosage form, dosage regimen and indications as the (5-methyl-2-oxo-1,3-dioxolen-4-yl) methyl ester is the same as the free acid or pharmaceutically accepted salt or ester forms already published. The advantage of the ester group yields enhanced blood levels of the active compound in the human or animal being treated.

Example 2
Preparation of (4-methyl-1,3-dioxol-2-one-5-yl-methyl) ester of methyldopa
Step A.
(S)N-(t-Butylcarbonyl)-3-hydroxy-α-methyl-tyrosine

A mixture of (S) 3-hydroxy-α-methyltyrosine sesquihydrate [methyldopa] (5.0 g, 21 mmol), triethylamine (2.1 g, 21 mmol) and di t-butyl dicarbonate (4.9 g, 23 mmol) in dimethylformamide, 50 mL, is stirred at 20—25°C for 1 hour and then at 60°C for 18 hours under nitrogen. After removing most of the dimethylformamide at 50—60°C and 0.1 mm, the residue is partitioned between a 5% citric acid solution, 25 mL, and ethyl acetate, 75 mL. The ethyl acetate extract is washed with two 10 mL portions of water saturated with sodium chloride, dried over anhydrous sodium sulfate, filtered and concentrated at 40°C and 15 mm. The residue is dried further at 20—25°C and 0.1 mm to give 6.5 g of the N-t-BOC derivative of methyldopa.

Step B.
4-Methyl-1,3-dioxol-2-one-5-ylmethyl(*S*)N-(t-Butoxycarbonyl)-3-hydroxy-α-methyltyrosinate

A solution of N-(t-butoxycarbonyl)-3-hydroxy-α-methyltyrosine (760 mg, 2.44 mmol), triethylamine (263 mg, 2.60 mmol) and 4-bromomethyl-5-methyl-1,3-dioxol-2-one (500 mg, 2.60 mmol) in dimethylformamide, 10 mL, is stirred at 60°C for 3 hours under nitrogen. Most of the dimethylformamide is removed at 50—60°C and 0.1 mm and the residue is partitioned between saturated sodium bicarbonate solution, 10 mL, and ethyl acetate, 50 mL. The ethyl acetate extract is washed with water saturated with sodium sulfate, filtered and concentrated at 40—45°C and 15 mm. Flash chromatography of the residue over 40 g of silica gel 60 (230—400 mesh) with 3% methanol-97% chloroform as eluent affords 320 mg of the desired ester.

Step C.
4-Methyl-1,3-dioxol-2-one-5-ylmethyl(*S*)3-Hydroxy-α-methyltyrosinate (2R, 3R) hydrogentartrate hemihydrate

A solution of 4-methyl-1,3-dioxol-2-one-5-ylmethyl (*S*)N-(t-butoxycarbonyl)-3-hydroxy-α-methyltyrosinate (1.24 g, 2.93 mmol) in ethyl acetate, 40 mL is cooled in an ice bath and saturated with anhydrous hydrogen chloride for 10 min. The solution is allowed to warm slowly to 20—25°C over 1.5 hours and then concentrated at 30—40°C and 15 mm. The residue is treated with a saturated sodium bicarbonate solution, 10 mL, and the deblocked ester is extracted into ethyl acetate, 50 mL. The ethyl acetate extract is washed with water saturated with sodium chloride, 10 mL, dried over anhydrous sodium sulfate, filtered and concentrated at 40—45°C and 15 mm. The ester base is converted to the (2R, 3R) hydrogen tartrate hemihydrate salt, 370 mg, mp 123—128°C dec, with L-tartaric acid in a 95% ethanol-ethyl acetate solution.
Calc. for $C_{15}H_{17}NO_7.C_4H_6O_6.1/2\ H_2O$: C, 47.30; H, 5.01; N, 2.90
Found: C, 47.34; H, 5.05; N, 289

The compound (4-t-butyl-1,3-dioxol-2-one-5-yl)methyl ester of methyldopa, as well as other 4-lower alkyl compounds, can be prepared in a similar fashion using analogous reactants. An example follows:

Example 3

Preparation of 4-t-butyl-1,3-dioxol-2-one-5-ylmethyl (*S*)-3-hydroxy-α-methyl tyrosinate (2R, 3R) hydrogentartrate hemihydrate

Step A.

5-t-Butyl-4-methyl-1,3-dioxolen-2-one

Forty-four g of a 12.5% solution of phosgene in toluene is added over 30 min to a well stirred solution of 4,4-dimethyl-2-hydroxy-3-pentanone (4.85 g, 37.3 mmol) in toluene, 90 ml, at 0—5°C. Following this addition, a solution of pyridine, 11.4 ml, in toluene, 25 ml, is then added to the cooled reaction mixture over 30 min. After stirring at 20—25°C for 20 hours, the reaction mixture is washed with 3N HCl and water saturated with NaCl. The toluene extract is dried over anhydrous sodium sulfate, filtered and concentrated under reduced pressure. After addition of p-toluenesulfonic acid hydrate, 100 mg, to the residue, it is stirred neat at 160°C for 24 hours. The resulting black liquid is dissolved in ethyl acetate, washed with a saturated sodium bicarbonate solution and a saturated sodium chloride solution and dried over anhydrous sodium sulfate. After filtering and concentrating under reduced pressure, the residue is distilled to give 3.5 g of product, bp 82—4°C at 3 mm.

Step B.

4-Bromomethyl-5-t-butyl-1,3-dioxolen-2-one

A mixture of 4-methyl-5-t-butyl-1,3-dioxolen-2-one (3.5 g, 22.4 mmol), N-bromosuccinimide (4.16 g, 23.4 mmol) and dibenzoylperoxide (20 mg) in carbon tetrachloride (100 ml) is stirred at reflux for one hour. After filtering, the filtrate is concentrated under reduced pressure to give the 4-bromomethyl derivative as an oil.

Step C.

4-t-Butyl-1,3-dioxol-2-one-5-ylmethyl (*S*)3-hydroxy-α-methyltyrosinate (2R,3R) hydrogentartrate hemihydrate

A mixture of 4-bromomethyl-5-t-butyl-1,3-dioxolen-2-one (5.27 g, 22.4 mmol) and (*S*)3-hydroxy-α-methyltyrosine sesquihydrate (5.3 g, 22.4 mmol) in dry dimethylformamide (35 ml) is stirred at 20—25°C for three hours under nitrogen. Solvent is removed at 40—50°C and 0.1—0.3 mm pressure and the residue is partitioned between a saturated aqueous sodium bicarbonate solution and ethyl acetate. The ethyl acetate extract is washed with water saturated with sodium chloride, dried over anhydrous sodium sulfate, filtered and concentrated. The crude ester is converted to the (2R, 3R) hydrogen tartrate salt and recrystallized from an 95% ethanol-ethyl acetatehexane mixture to give 6.75 g (57.4%) of the desired ester as the hydrogentartrate hemihydrate salt, mp=113—121°C, slow decomposition.

Anal. Calcd. for $C_{18}H_{23}NO_7.C_4H_6O_6.1/2\ H_2O$: C 50.38, H, 5.77, N, 2.67
Found: C, 50.40, H, 5.85; N, 2.54

**Claims for the contracting states: BE, CH, DE, FR, GB, IT, LI, LU, NL, SE**

1. The (5-alkyl-2-oxo-1,3-dioxolen-4-yl)methyl ester of a carboxylic acid wherein alkyl has a 1—6 carbon atoms and wherein the carboxylic acid is cefoxitin, methyldopa or 2-(ethylthio)-6β-(1R-hydroxyethyl)-penem-3-carboxylic acid.

2. The compound 4-t-butyl-1,3-dioxol-2-one-5-ylmethyl(S)-3-hydroxy-α-methyltyrosinate (2R, 3R) hydrogentartrate, and that being in the hemihydrate form.

3. The compound 4-methyl-1,3-dioxol-2-one-5-ylmethyl(S)-3-hydroxy-α-methyltyrosinate (2R, 3R) hydrogentartrate, and that being in the hemihydrate form.

4. A pharmaceutical composition containing an effective amount of the compound of claims 1—3 in a pharmaceutically acceptable carrier.

**Claims for the contracting state: AT**

1. A process for preparing the (5-alkyl-2-oxo-1,3-dioxolen-4-yl)methyl ester of a carboxylic acid wherein alkyl has 1—6 carbon atoms and wherein the carboxylic acid is cefoxitin, methyldopa or 2-(ethylthio)-6β-(1R-hydroxyethyl)-penem-3-carboxylic acid, comprising reacting a compound having the formula

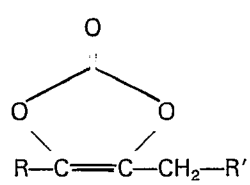

wherein R is alkyl of 1—6 carbon atoms and R' is OH or halogen with the free acid or a salt form of cefoxitin, methyldopa or 2-(ethylthio)-6β-(1R-hydroxyethyl)-penem-3-carboxylic acid.

2. The process of claim 1 wherein R' is Br or Cl and the carboxylic acid is in the salt form, preferably the lithium or silver salt.

3. The process of claims 1 or 2 for preparing 4-t-butyl-1,3-dioxol-2-one-5-ylmethyl(S)-3-hydroxy-α-methyltyrosinate (2R, 3R) hydrogentartrate, and that being in the hemihydrate form.

4. The process of claims 1 or 2 for preparing 4-methyl-1,3-dioxol-2-one-5-ylmethyl(S)-3-hydroxy-α-methyltyrosinate (2R, 3R) hydrogentartrate, and that being in the hemihydrate form.

**Patentansprüche für die Vertragsstaaten: BE, CH, DE, FR, GB, IT, LI, LU, NL, SE**

1. Der (5-Alkyl-2-oxo-1,3-dioxolen-4-yl)methylester einer Carbonsäure, worin Alkyl 1 bis 6 Kohlenstoffatome hat, und worin die Carbonsäure Cefoxitin, Methyldopa oder 2-(Ethyl-thio)-6β-(1R-hydroxyethyl)-penem-3-carbonsäure ist.

2. Die Verbindung 4-tert.Butyl-1,3-dioxol-2-on-5-ylmethyl(S)-3-hydroxy-α-methyltyrosinat-(2R,3R)-hydrogentartrat in der Hemihydratform.

3. Die Verbindung 4-Methyl-1,3-dioxol-2-on-5-ylmethyl(S)-3-hydroxy-α-methyltyrosinat-(2R,3R)-hydrogentartrat in der Hemihydratform.

4. Eine pharmazeutische Zusammensetzung, enthaltend eine wirksame Menge der Verbindung der Ansprüche 1 bis 3 in einem pharmazeutisch annehmbaren Träger.

**Patentansprüche für den Vertragsstaat: AT**

1. Ein Verfahren zur Herstellung des (5-Alkyl-2-oxo-1,3-dioxolen-4-yl)methylesters einer Carbonsäure, worin Alkyl 1 bis 6 Kohlenstoffatome hat, und worin die Carbonsäure Cefoxitin, Methyldopa oder 2-(Ethylthio)-6β-(1R-hydroxyethyl)-penem-3-carbonsäure ist, umfassend die Umsetzung einer Verbindung der Formel

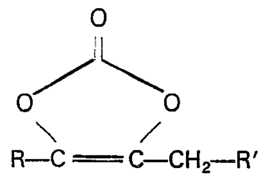

worin R Alkyl mit 1 bis 6 Kohlenstoffatomen ist und R' OH oder Halogen ist, mit der freien Säure oder einer Salzform von Cefoxitin, Methyldopa oder 2-(Ethylthio)-6β-(1R-hydroxyethyl)-penem-3-carbonsäure.

2. Das Verfahren des Anspruchs 1, worin R' Br oder Cl ist, und die Carbonsäure in der Salzform, vorzugsweise als Lithium- oder Silbersalz, eingesetzt wird.

3. Das Verfahren des Anspruchs 1 oder 2 zur Herstellung von 4-tert.-Butyl-1,3-dioxol-2-on-5-ylmethyl(S)-3-hydroxy-α-methyltyrosinat-(2R,3R)-hydrogentartrat in der Hemihydratform.

4. Das Verfahren des Anspruchs 1 oder 2 zur Herstellung von 4-Methyl-1,3-dioxol-2-on-5-ylmethyl(S)-3-hydroxy-α-methyltyrosinat-(2R,3R)-hydrogentartrat in der Hemihydratform.

**Revendications pour les Etats Contractants: BE, CH, DE, FR, GB, IT, LI, LU, NL, SE**

1. Ester (5-alkyl-2-oxo-1,3-dioxolen-4-yl)-méthylique d'un acide carboxylique dans lequel et radical alkyle contient 1 à 6 atomes de carbone et dans lequel l'acide carboxylique est la cefoxitine, le méthyldopa ou l'acide 2-(éthylthio)-6β-(1R-hydroxyéthyl)-penem-3-carboxylique.

2. Composé hydrogéno-tartrate (2R,3R) de 4-t-butyl-1,3-dioxol-2-one-5-ylméthyl(S)-3-hydroxy-α-méthyltyrosinate, et celui-ci étant sous la forme hémihydratée.

3. Composée hydrogéno-tartrate (2R,3R) de 4-méthyl-1,3-dioxol-2-one-5-ylméthyl(S)-3-hydroxy-α-méthyltyrosinate, et celui-ci étant sous la forme hémihydratée.

4. Composition pharmaceutique contenant une proportion efficace du composé selon les revendications 1 à 3 dans un véhicule pharmaceutiquement acceptable.

**Revendications pour l'Etat Contractant: AT**

1. Procédé de préparation d'ester (5-alkyl-2-oxo-1,3-dioxolen-4-yl)-méthylique d'un acide carboxylique dans lequel le radical alkyle contient 1 à 6 atomes de carbone, et dans lequel l'acide carboxylique est la cefoxitine, le méthlydopa ou l'acide 2-(éthylthio)-6β-(1R-hydroxyéthyl)-penem-3-carboxylique, qui comprend la réaction d'un composé de formule:

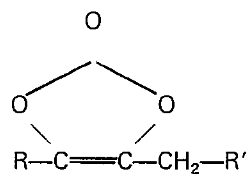

dans laquelle R est un radical alkyle de 1 à 6 atomes de carbone et R' est un radical OH ou halogène avec l'acide libre ou un sel de cefoxitine, méthyldopa ou acide 2-(éthylthio)-6β-(1R-hydroxyéthyl)-penem-3-carboxylique.

2. Procédé selon la revendication 1, dans lequel R' est Br ou Cl et l'acide carboxylique est sous forme d'un sel, de préférence, le sel de lithium ou d'argent.

3. Procédé selon les revendications 1 ou 2, pour préparer l'hydrogèno-tartrate (2R, 3R) de 4-t-butyl-1,3-dioxol-2-one-5-ylméthyl(S)-3-hydroxy-α-méthyltyrosinate et celui-ci étant sous la forme hémihydratée.

4. Procédé selon la revendication 1 ou 2, pour préparer l'hydrogéno-tartrate (2R,3R) de 4-méthyl-1,3-dioxol-2-one-5-ylméthyl(S)-3-hydroxy-α-méthyltyrosinate et celui-ci étant sous la forme hémihydratée.